# EUROPEAN PATENT APPLICATION

(11) **EP 4 026 494 A1**
(43) Date of publication of application: **13.07.2022**
(21) Application number: 21150419.6
(22) Date of filing: 06.01.2021
(51) Int. Cl.: A61B 5/06, A61B 5/00, A61B 34/20, A61B 5/107

(54) **TRACKING THE POSITION OF AN INTERVENTIONAL DEVICE**

(71) Applicant: Koninklijke Philips N.V., 5656 AG Eindhoven (NL)
(72) Inventor: GIJSBERS, Gerardus Henricus Maria, 5656 AE Eindhoven (NL); WILDEBOER, Rogier Rudolf, 5656 AE Eindhoven (NL); CLUITMANS, Matthijs Joseph Maria, 5656 AE Eindhoven (NL); KOLEN, Alexander Franciscus, 5656 AE Eindhoven (NL); RUETTEN, Walter, 5656 AE Eindhoven (NL); KAHLMAN, Josephus Arnoldus Johannes Maria, 5656 AE Eindhoven (NL)
(74) Representative: Philips Intellectual Property & Standards

(57) **Abstract**

A mechanism for adjusting the direction of one or more crossing electric fields that have been induced by a set of electrodes positioned on a patient, without necessitating movement of any of the electrodes. This is achieved through the use of one or more electrode patches, which effectively allow an electric field generator to control a position of a source/sink of an electric field, to thereby control a direction of the electric field.

## Description

### FIELD OF THE INVENTION

The present invention relates to the field of interventional devices, and in particular, to the field of tracking the position of (electrodes of) an interventional device.

### BACKGROUND OF THE INVENTION

There is an increasing interest in mechanisms for tracking a position of an interventional device within a subject, i.e. an individual such as a person or animal, where an interventional device is any medical device designed to access an internal part of the subject (e.g. catheters, surgical instruments and the like).

One area in which position tracking is of particular benefit is in a dielectric imaging process, sometimes referred to as electroanatomical mapping process. In a dielectric imaging process, two or more crossing electrical fields are induced by a set of electrodes positioned on the outside of the subject ("external electrodes"), such as on the surface of the subject. These electric fields induce position dependent responses, such as a voltage response, in electrodes placed within the body (an "internal electrode"). The position of an internal electrode can thereby be tracked by monitoring the response of the electrode to these electric fields. By tracking and recording the position of the internal electrode(s), a model of the internal anatomy of the subject can be (re)constructed. This is because it can be assumed that the internal electrodes will only be located within cavities of the subject, thereby allowing the bounds of the cavity to be constructed.

One example of a suitable mechanism for constructing a model of the internal anatomy of the subject based on the response of internal electrodes to crossing electric fields induced by external electrodes is disclosed by the European Patent Application having publication number EP 3568068 A1.

There is an ongoing desire to facilitate improved tracking of the position of (electrodes of) an interventional device, particularly for dielectric imaging processes in which accurate tracking of the position of the internal electrodes means a more accurate model of the internal anatomy can be constructed.

### SUMMARY OF THE INVENTION

The invention is defined by the claims.

According to example in accordance with an aspect of the invention, there is provided an electrode patch configured for use with an electric field generating arrangement for inducing two or more crossing electric fields within a subject for use in tracking the position of an interventional device within the subject.

The electrode patch comprises two or more electrically responsive portions that are individually addressable by an electric field generator of the electric field generating arrangement, so that, when the electrode patch is positioned on the surface of the subject, a direction of an electric field between the electrode patch and one other electrode positioned on the surface of the subject is adjustable.

An underlying recognition of the present disclosure is that localization of an internal electrode, such as an electrode on a catheter, is improved if the electric fields generated by an external electrode set/array are oriented in a correct/desired orientation, e.g. if they are orthogonal to one another. The disclosure also recognizes that positioning external electrodes, so that generated electric fields align with a desired orientation, e.g. are orthogonal to one another, is a difficult and time-consuming task, and that, even if initially positioned appropriately, the direction of an electric field may change over time (e.g. if the subject moves or shifts, or during a respiratory and/or circulatory cycle of the subject). Where sterility is important, it is desirable to avoid unnecessary repositioning of external electrodes on the subject. It is also recognized that repositioning of external electrodes takes time, which could impact on the efficiency of treatment and/or diagnosis being performed on the subject (with the aid of the crossing electric fields).

This disclosure proposes an electrode patch that makes use of a new approach for defining the position of the electric fields, to provide a level of control over the direction of the electric fields when the external electrodes have been positioned on the subject, without needing to remove and reposition the external electrodes. This is achieved through the use of one or more such electrode patches, which allow the position of a source/sink of an electric field to be changed without movement of the electrode patch, through the use of separately addressable electrically responsive portions of the electrode patch.

In particular, the electric field generator is able to control which element(s) (e.g. part or parts) of the electrode patch(es) is/are used to generate/receive an electric field, to thereby control the direction of the electric field.

As previously explained, the use of the proposed electrode patch means that the direction of at least one of the electric fields can be changed without needing to move the set/array of electrodes (used to generate the crossing electric fields). Controlling the direction of the electric field(s) further allows for control over the orthogonality of the electric fields. The more orthogonal the crossing electric fields, the greater the accuracy in tracking the internal electrodes.

In some examples, all electrically responsive portions of the electrode patch are addressable, by the electric field generator, with an adjustable current amplitude or phase, i.e. to contribute to the generation of an electric field.

In some embodiments, each electrode patch comprises an array of sub-electrodes electrically isolated from one another. Optionally, each electrically responsive portion comprises a different set, or sub-set, of one or more of the sub-electrodes.

In some examples, each electrode patch comprises a sheet of electrically responsive material, wherein each electrically responsive portions is able to overlap another electrically responsive portion. The sheet of electrically responsive material may have a relatively low conductivity, so that providing a voltage to a first portion of the sheet results in the origin/sink of an electric field (generated by the sheet) being in a different position to providing a voltage to a second, different portion of the sheet.

There is also proposed a set of external electrodes for positioning on a surface of the subject, the set of external electrodes comprising at least one previously described electrode patch. The set of electrode patches thereby facilitate, when positioned on a subject and through appropriate control by an electric field generator, the generation of a plurality of crossing electric fields, in which the direction of at least one of these electric fields is adjustable.

The at least one electrode patch preferably comprises a plurality of electrode patches, for instance, at least one electrode patch comprises at least six electrode patches. Each electrode patch is embodied as previously described electrode patches. The greater the number of electrode patches in the set of external electrodes, the greater control over the direction and orthogonality of the crossing electric fields.

There is also proposed an electric field generator for use with an electric field generating arrangement for inducing two or more crossing electric fields within a subject for use in tracking the position of an interventional device within the subject.

The electric field generator is configured to control an electric signal provided to (each of) a set of external electrodes, as previously described, to thereby, when the set of external electrodes is positioned on the surface of the subject, induce at least two crossing electric fields within the subject.

The electric field generator is thereby configured to control electric signals provided to each external electrode in a set of external electrodes positioned on a surface of the subject to thereby induce at least two crossing electric fields within the subject. Here, the set of external electrodes comprises at least one electrode patch, each electrode patch comprising two or more electrically responsive portions that are individually addressable so that, when the set of external electrodes is positioned on the surface of the subject, a direction of an electric field between the electrode patch and one other external electrode is adjustable.

The electric field generator thereby that works together with the set of external electrodes to provide the crossing electric fields, so that the set of external electrodes and the electric field generator are a set of interrelated products.

The electric field generator may be operable in two or more different control configurations, wherein each control configuration defines which of the electrically responsive portions of each electrode patch is addressed by the electric field generator (e.g. provided with a non-zero voltage/current), so that the direction of one or more of the at least two crossing electric fields differs in different control configurations.

In other words, the electric fields generator is configured to control which of the electrically responsive portions are "active", i.e. which electrically responsive portions contribute to defining the position of the electric fields (e.g. which parts generate and/or receive an electric field). Different sets of (one or more) electrically responsive portions are active in different control configurations.

The electric field generator may be further configured to determine a measure of orthogonality of the at least two electric fields for one or more different control configurations of the set of external electrodes. A measure of orthogonality indicates how close the at least two electric fields are to being orthogonal to one another. For instance, a measure of orthogonality may change responsive to a change in angle between the crossing electric fields, so that a determination can be made as to whether the electric fields are orthogonal to one another.

The electric field generator may be configured to determine an orthogonality of the at least two electric fields by: obtaining, from one or more electrodes responsive to the at least two crossing electric fields, an electrical response of the one or more electrodes to the at least two crossing electric fields; and determining, based on the electrical response of the one or more electrodes, a measure of orthogonality of the at least two crossing electric fields.

The method of controlling the direction of an electrical field can also be applied in a situation where there is only one electrical field between an electrode pair, which can be used to, for example, determine a position of an electrode or a set of electrodes on a device in one (i.e. a single) direction, for instance, in a vessel or other tubular organ in the body. By controlling the electrical field direction it can be aligned with the orientation of the tube.

Thus, in some aspects of the inventive concept, the set of external electrodes and the electric field generator may be configured to only generate a single electric field, having an adjustable direction through use of the electrode patch herein disclosed. Thus, the term "two crossing electric fields" may be replaced, where appropriate, by the term "single electric field" to achieve another aspect of the inventive concept.

Mechanisms for determining a measure of orthogonality based on the electrical response of electrodes to crossing electric fields are well established in the art, and may employ approaches such as checking or determining weights in a whitening transformation, checking or determining the correlation between recordings of two or more electrical fields, or checking or determining the angle or average angle between the direction of maximum gradient in each crossing field.

The electric field generator may be configured to: determine a measure of orthogonality of the at least two crossing electric fields for each of a plurality of different control configurations; and identify the control configuration associated with the greatest orthogonality (e.g. provides crossing electric fields that are closest to orthogonality) based on the determined measures of orthogonality.

The at least two crossing electric fields may comprise at least two crossing electric fields of different frequencies. This facilitates ease of identification of the relative position and/or location of an (internal) electrode within the crossing electrode fields, as the response of the electrode to the cross electric fields will be different at different distances from the source/sink of each electric field, thereby allowing or enabling triangulation of the (internal) electrode within the crossing electric fields.

According to examples in accordance with an aspect of the invention, there is provided an electric field generating arrangement for inducing two or more crossing electric fields within a subject for use in tracking the position of an interventional device within the subj ect.

The electric field generating arrangement comprises a set of external electrodes as previously described and an electric field generator as previously described.

Thus, the electric field generating arrangement comprises: a set of external electrodes for positioning on a surface of the subject; and an electric field generator configured to control an electric signal provided to each external electrode in the set of external electrodes to thereby, when the set of external electrodes is positioned on the surface of the subject, induce at least two crossing electric fields within the subject, wherein the set of external electrodes comprises at least one electrode patch, each electrode patch comprising two or more electrically responsive portions that are individually addressable by the electric field generator so that, when the set of external electrodes is positioned on the surface of the subject, a direction of an electric field between the electrode patch and one other external electrode is adjustable.

Preferably, the at least two crossing electric fields comprises three crossing electric fields. This embodiment facilitates identification of a 3D location of an electrode within the crossing electric fields.

There is also proposed a device tracking arrangement comprising: the electric field generating arrangement previously described; and a device tracking system configured to: obtain, at a first input and from an internal electrode positioned upon an interventional device within the subject, an electrical response of the internal electrode to the at least two crossing electric fields; and determine, based on the electrical response of the internal electrode, a relative position of the interventional device within the subject.

There is also proposed a computer-implemented method for inducing two or more crossing electric fields within a subject for use in tracking the position of an interventional device within the subject, the computer-implemented method comprising: controlling voltages provided to each external electrode in a set of external electrodes positioned on a surface of the subject to thereby induce at least two crossing electric fields within the subject, wherein the set of external electrodes comprises at least one electrode patch, each electrode patch comprising two or more electrically responsive portions that are individually addressable so that, when the set of external electrodes is positioned on the surface of the subject, a direction of an electric field between the electrode patch and one other external electrode is adjustable.

This computer-implemented method is effectively the process performed by the electric field generator. The skilled person would be readily capable of modifying this method to include and step performed by the electric field generator, and modify the electric field generator to carry out any step of any herein disclosed method.

There is also proposed a computer program product comprising computer program code means which, when executed on a computing device having a processing system, cause the processing system to perform all of the steps of the any herein described method.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates positions for a set of external electrodes positioned on a subj ect;
Figure 2 illustrates a co-ordinate system for defining a direction of electric fields generated by the external electrodes;
Figure 3 illustrates a device tracking arrangement;
Figure 4 illustrates an approach for reconstructing an anatomical model from a point cloud;
Figure 5 illustrates an electrode patch for use in/as an embodiment;
Figure 6 illustrates a use case scenario for a set of electrode patches;
Figure 7 illustrates an electrode patch for use in/as another embodiment;
Figure 8 illustrates an electrode patch for use in/as yet another embodiment;
Figure 9 illustrates an electrode patch for use in/as another embodiment;
Figure 10 illustrates a method for use in an embodiment; and
Figure 11 illustrates a method for use in another embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for adjusting the direction of one or more crossing electric fields that have been induced by a set of electrodes positioned on a patient, without necessitating movement of any of the electrodes. This is achieved through the use of one or more electrode patches, which effectively allow an electric field generator to control a position of a source/sink of an electric field, to thereby control a direction of the electric field.

Embodiments are based on the realization that the direction and/or orientation of electric fields can be changed by controlling which of a plurality of electrically responsive portions of an electrode patch contribute to the generation of the electric field. This means that the direction of the electric field can be changed dynamically, and without necessitating the movement of any electrodes on the patient/subject.

Proposed concepts can be employed in any suitable device tracking system that makes use of electric fields, induced in the subject, to track the location of an electrode and/or interventional device within the subject. One example system is the Philips' KODEX-EPD system for performing imaging of an anatomical cavity of a subject.

For the purposes of contextual understanding, the principle and purpose of inducing electric fields within a subject will be hereafter described.

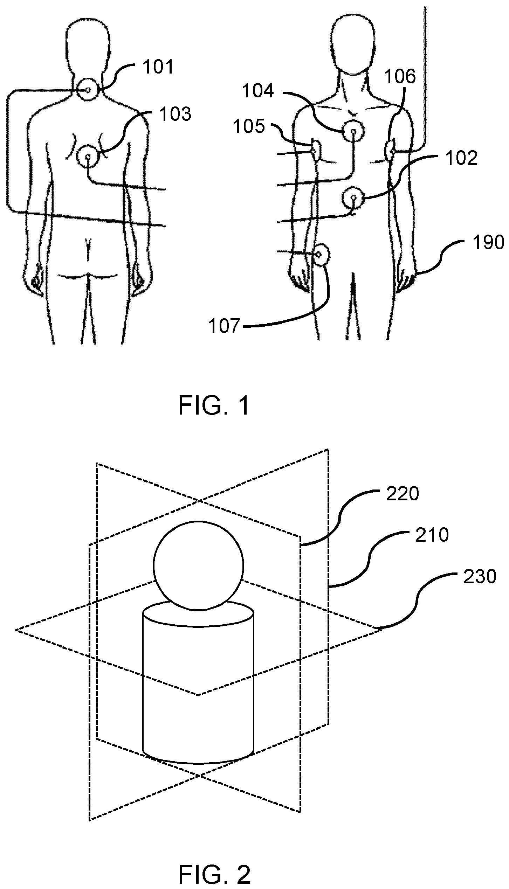

Figure 1 illustrates positions for a set of external electrodes positioned on a subject 190. External electrodes are electrodes that are positioned externally to a subject, and are contrasted with internal electrodes that are positionable within the subject.

The illustrated set of external electrodes comprises a first external electrode 101, a second external electrode 102, a third external electrode 103, a fourth external electrode 104, a fifth external electrode 105 and a sixth external electrode 106. The illustrated set further comprises a reference electrode 107, which can act as a "ground" for defining a base/background level of electric field activity in the subject.

An electric signal provided to each external electrode is controlled to thereby define crossing electric fields between the electrodes. In particular, the first 101 and second 102 external electrodes form a first pair of electrodes ("electrode pair"), and signals provided to the first electrode pair can be controlled to induce a first electric field therebetween. The third 103 and fourth 104 electrodes form a second pair of electrodes, and signals provided to the second electrode pair can be controlled to induce a second electric field therebetween. The fifth 105 and sixth 106 electrodes form a third pair of electrodes, and signals provided to the third electrode pair can be controlled to induce a third electric field therebetween.

Electric signals provided to each external electrode, and in particular to each pair of electrodes, can control the frequency and/or magnitude/strength of the crossing electric fields. The electric signals supplied to the electrodes may be controlled by an electric field generator (not illustrated in Figure 1).

The crossing electric fields are usable to track or identify a location of electrodes positioned within the crossing electric fields. In particular, a response of an electrode to each electric field will change as a position within the electric field changes (e.g. a distance from a source/sink of the electric field changes). The response of an electrode to the crossing electric fields can be mapped or associated with a particular position within the electric fields.

In other words, an electrical response of the internal electrode to the crossing electric fields can be processed to determine a relative position of the internal electrode (e.g. and therefore of any interventional device comprising the internal electrode) within the subj ect.

In particular, each electric field may be controlled to have a particular/different frequency. This facilitates determination of the relative position of an electrode with respect to each electric field, by assessing the response of the electrode to the particular frequency of the electric field. This information can be used to effectively triangulate/trilaterate the position of the electrode with respect to a co-ordinate system defined by the electric fields.

One of the electrode pairs may be omitted, e.g. if only two crossing electric fields are desired (e.g. for performing a 2-dimensional tracking process).

Figure 2 schematically illustrates a co-ordinate system for defining a direction of electric fields generated by the external electrodes. The co-ordinate system defines three cardinal planes 210, 220, 230. The crossing electric fields may, for instance, be optimally positioned when the direction of each electric field is parallel to a respective cardinal place 210, 220, 230.

The intent of the crossing electric fields, e.g. such as those generated using the set illustrated by Figure 1, is to facilitate identification of the position of an electrode (or group of electrodes) with respect to a co-ordinate system, such as that illustrated by Figure 2.

A more complete example of how to track the position of an internal electrode with respect to crossing electric fields (i.e. within the subject), and optionally how to further exploit this information, e.g. for the construction of an anatomical model of an anatomical cavity of the subject, is hereafter described for the purposes of improved contextual understanding.

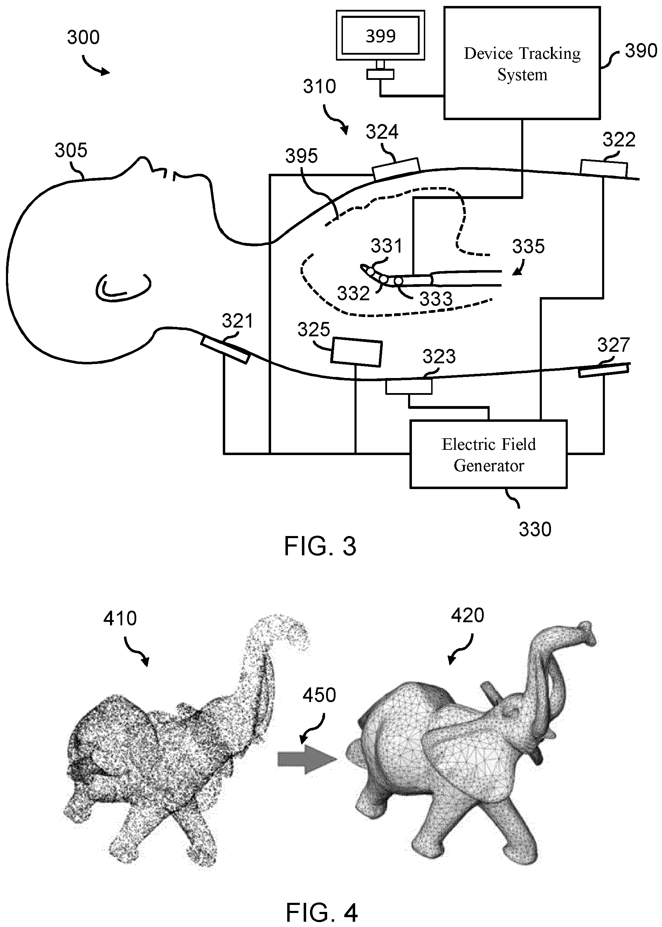

Figure 3 conceptually illustrates a device tracking arrangement 300 for tracking the position of an interventional device within a subject 305.

The device tracking arrangement 300 may also be configured to generate an anatomical model (of an anatomical cavity, such as a blood vessel and/or heart chamber) using for example a dielectric imaging process or electroanatomical mapping process. The present disclosed solutions may have an advantageous effect on such processes, but such process are not essential for the present solutions to have their advantageous effect..

The device tracking arrangement 300 comprise an electric field generating arrangement 310 and a device tracking system 390. The electric field generating arrangement is itself an embodiment of the invention. The electric field generator may be a unit separate from the device tracking system but may also be part of it and/or integrated in it.

The electric field generating arrangement 310 comprises a set of external electrodes 331. 332. 333, 324, 325, 327 for positioning externally with respect to the subject 305 (e.g. as electrode patches provided on a skin of the subject). The set 310 of external electrodes may comprise a plurality of electrode pairs angled with respect to one another (e.g. the imaginary lines connecting the electrodes of a pair being oriented orthogonally to one another) due to the positioning of the pairs electrodes on the body, so that any electric fields generated by the electrode pairs are angled with respect to one another. These electrode pairs may comprise a first electrode pair (formed of a first 321 and second 322 external electrode), a second electrode pair (formed of a third 323 and fourth 324 external electrode) and a third electrode pair (formed of a fifth 325 and sixth (not visible) external electrode). One of more of these electrode pairs may be omitted. The set of external electrodes may also comprise a reference electrode 327. This may be e.g. be a leg electrode or other extremity electrode.

Thus, the external electrodes are placeable in a similar manner to the set of external electrodes illustrated in Figure 1.

The electric field generating arrangement 310 also comprises an electric field generator 330, which is adapted to control (characteristics of) an electric signal (e.g. voltage and/or current) supplied to each external electrode.

The electric field generating arrangement 310 is configured to generate a plurality of (here: three) crossing (intra-body) electrical fields using the external electrodes. This is performed using appropriate control of the electric signals provided to each external electrode.

In particular, each electrode pair may be appropriately controlled to induce an electric field between each electric pair. Thus, where there are three electrode pairs, three electric fields may be generated. Preferably, the frequency of each generated electric field is controlled to be different, to facilitate increased ease in identifying a relative location of an electrode positioning within the crossing electric fields.

The generated electric fields can be used to define or establish a relative location of an (internal) electrode positioned within the crossing electric fields. In particular, as previously explained, the (electrical) response of an internal electrode to the electric fields changes as the relative position of the internal electrodes moves about (within) the subject. This is at least partly because the induced electrical fields' distribution is inherently inhomogeneous due to the different dielectric properties and absorption rates (related to conductivity) of the interrogated tissues. The principle of crossing electric fields thereby facilitates tracking of the relative position of the internal electrode using a device tracking system 390 that monitors a response of any internal electrodes to the crossing electric fields, e.g. by mapping the (electrical) response of an electrode to a relative position within the subj ect.

By way of further explanation, for the purposes of improved conceptual understanding, if the crossing electric fields are controlled to have a different frequency with respect to one another, then the response of the internal electrode to each frequency could be used to determine a relative distance between from each source/sink of the corresponding electric field. This principle can be used to effectively triangulate the relative position of the internal electrode within the crossing electric fields.

For instance, if there are three external electrode pairs, positioned to emit electric fields of different frequencies (E₁, E₂, E₃) that are angled (e.g. near-orthogonal) with respect to one another, a voltage response (V₁, V₂, V₃) of an internal electrode (identifying a voltage (e.g. between the electrode and the reference electrode or between the electrode and the electrode generating the electric field) at each of these three frequencies) will differ depending upon position within the anatomical cavity.

Other forms of response for an internal electrode (e.g. an impedance response or a capacitive, e.g. indicating change in impedance/capacitance between the internal electrode and an external electrode) will be apparent to the skilled person.

The electric field generator may be configured to control the electric fields, generated using the set of external electrodes, to operate in the frequency range of e.g.30-100 kHz. This frequency range is particularly useful for ensuring penetration of a subject, whilst providing a frequency range that attenuates in human tissue without significant damage/injury to the tissue. The reference electrode serves as an electric reference for all measurements taken by electrodes, e.g. as a reference for the response of the internal electrodes.

As previously explained, the response of an internal electrode may be used to track the location of the internal electrode (and/or an interventional device upon which the internal electrodes are mounted). This information can be used to construct an anatomical model of an anatomical cavity (i.e. a cavity in which the interventional device is able to move).

The operation of the device tracking system 390 for performing a process of constructing of an anatomical model of an anatomical cavity is hereafter described. Other descriptions and/or embodiments of this imaging process are disclosed by the European Patent Application EP 0775466 A2, EP 3568068 A1 and EP 3607879 A1.

Broadly, the device tracking system 390 may build an anatomical model of an anatomical cavity 395 (e.g. a chamber, vessel or void) within a subject by measuring the potential of crossing electric fields by internal electrodes (i.e. the influence of the electric fields on the internal electrodes) positioned within the subject that are transformed in relative positions. This allows the boundaries, border or perimeter of anatomical cavity to be mapped, thereby building a point cloud of the anatomical cavity.

Thus, the device tracking system makes use of a plurality of internal electrodes 331. 332. 333 to be positioned within the anatomical cavity (e.g. electrodes positioned on an interventional device 335 to be inserted into the anatomical cavity). A distance between each of the internal electrodes may be predetermined and/or known.

The device tracking system 390 is configured to receive (and optionally provide) signals from/to the internal electrodes 331. 332. 333 to determine a response of the internal electrodes to the crossing electric fields.

In particular, to perform an imaging process, the response of the internal electrodes (e.g. to externally applied electric fields by the external electrodes) is iteratively recorded. The imaging processor repeatedly defines/updates and applies a transfer function ("V2R function") that transforms each recorded response to Euclidian coordinates (R-space), whilst ensuring known properties of the internal electrodes and/or interventional device 335 (e.g. electrode spacing and electrical weight length) as well as a set of other constraints are maintained. The transfer function can effectively learn and linearize the distorted relation between the measured responses and the actual position in three dimensions by taking the known inter-electrode distances as a reference. By way of example only, if an internal electrode has a response matching a previously measured response then the relative distance to the responses measured by the other internal electrodes can be determined and used to improve the transfer function.

In this way, an R-space cloud of points (known Euclidian co-ordinates) can be built up and updated as the internal electrodes are moved within the anatomical cavity. Using the updated R-space cloud of points, a reconstruction algorithm generates an anatomical model of the anatomical cavity. The anatomical model may, for example, be a 3D surface that depicts or models the (bounds of) the anatomical cavity.

The process of reconstructing an anatomical model from a point cloud is conceptually illustrated in Figure 4, which demonstrates a process 450 in which a cloud of R-space points 410 ("point cloud") is transformed into an anatomical model 420. In the illustrated example, this is performed by creating a (3D) surface from the point cloud data, methods of which will be readily apparent to the skilled person.

For example, a point cloud can be converted into a polygon mesh or triangular mesh model (or other surface model) using a surface reconstruction approach. A variety of suitable approaches is discussed in Berger, Matthew, et al. "A survey of surface reconstruction from point clouds." Computer Graphics Forum. Vol. 26. No. 1. 2017, and further mechanisms will be readily apparent to the skilled person.

In this way, the "global field" measurements (i.e. the effect of the electric fields generated by external electrodes on the internal electrodes) can be used to generate an (approximate) anatomical model of the anatomical cavity.

Referring back to Figure 3, more precise identification of the bounds and features of the anatomical model can be performed by monitoring the response of the internal electrodes 331. 332. 333 to local electric fields (e.g. fields generated by other internal electrodes). Thus, in some embodiments, the device tracking system 390 may also control the internal electrodes 331. 332. 333 to generate an electric field (which can be detected by other internal electrodes). The response of the internal electrodes to local electric fields can be labelled "local field measurements", as compared to the response of the internal electrodes to externally induced electric fields which can be labelled "global field measurements".

For instance, changes in a local electrical field (being an electric field induced between two internal electrodes 331. 332. 333) can indicate the presence or absence of tissue between the two internal electrodes. A response of an internal electrode to a local electric field can therefore be used to identify the presence or absence of tissue, to thereby facilitate tuning or updating of the anatomical model.

In some examples, additional processing of global field measurements (i.e. the responses of the internal electrodes to electric fields induced by the external electrodes) can be performed to improve the precision of the bounds and features of the anatomical model.

For example, regions with inherently marked steep gradients in the electrical field responses can be identified. It is recognized that such regions indicate the bounds of the anatomical cavity and/or other information, e.g. drainage of vessels into or out of a cardiac chamber as well as the valves of a cardiac chamber. These features can thereby be uniquely identified by the system and imaged even without physically visiting them with the interventional device 335.

The combined global and local field measurements enable sophisticated detection and effective handling of inconsistencies and outliers, level of electrode shielding/coverage (e.g. by measuring location inter-correlation), pacing (saturation), as well as physiological drift. Drift can, for example, be detected using a moving window over time and corrected continuously whereby the internal electrode location remains accurate throughout the whole procedure making the system resilient to drift.

The above-provided description of an imaging process is only an example, and the skilled person would be readily capable of modifying the described process appropriately.

The skilled person will appreciate that the transfer function ("V2R algorithm") can also be used to track a location of the internal electrodes with respect to a constructed anatomical model. This facilitates the generation and display of an anatomical model and an indicator of a current location/position of an interventional device 335 mounting internal electrodes 331, 332, 333 with respect to the anatomical model.

Of course, a visual representation of any generated anatomical model and/or determined position may be generated and provided at a user interface 399.

The present disclosure relies upon the recognition that localization/tracking of internal electrodes, i.e. determining a relative position of the internal electrodes, is more accurate/precise when electric fields induced by the set of external electrodes are increasingly orthogonal to one another. In other words, the more orthogonal the fields (i.e. the closer to orthogonality) the more accurate precise the tracking or modelling may become.

However, it is herein recognized that placement of electrodes on the subject to create entirely orthogonal electric fields is not always possible, e.g. due to anatomical constraints or difficulty in accurately (and manually) placing the electrodes. Moreover, electric field orthogonality at the location at which the internal electrodes are located (e.g. at the heart when one or more parts of the heart is to be analyzed or treated) might be distorted by the varying dielectric properties of the tissues in between the electrodes. Thus, optimal geometrical orthogonality achieved during placement of electrodes does therefore not necessarily also result in optimal electric orthogonality achieved with such placement.

Although a simple approach to overcoming these issues could be to simply reposition the electrodes, this can introduce significant sterility problems (e.g. for a catheterized patient) and introduces inconvenience and stress for a clinician as well as time-inefficient treatments due to lengthy electrode placement optimization procedures.

The present disclosure proposes the use of one or more electrode patches for the external electrodes where at least one, but preferably more than one and most preferably, each electrode patch comprises two or more electrically responsive portions that are individually addressable by the electric field generator. This facilitates modification of the direction of an electric field generated using such multi portion electrode patch, by controlling which of the electrically responsive portions contribute to the electric field.

Thus, the orientation of one or more of the electric fields can be adjusted by selecting which element/portion contributes to the generation of a particular electric field. Adjusting the orientation/direction of the electric fields facilitates and/or enables control over the orthogonality of the crossing electrical fields, being a measure of how close the crossing electric fields are to being orthogonal to one another. Thus, the orthogonality measure changes as the crossing electric fields converge to and diverge from orthogonality.

Electrically responsive portions that contribute to the generation of an electric field (of the crossing electric fields) are labelled "active portions", with unused electrically responsive portions being labelled "inactive portions".

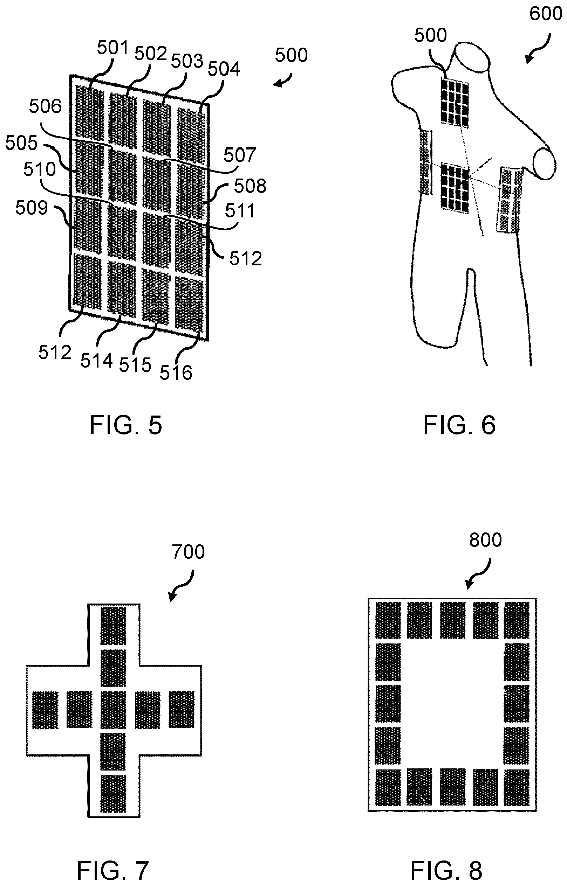

Figure 5 illustrates an exemplary electrode patch 500 for use in/as an embodiment of the invention.

The electrode patch 500 comprises a plurality of individual sub-electrodes 501-516, which are electrically isolated from one another, thus forming an "array patch". Each sub-electrode acts as a different electrically responsive portion of the electrode patch, and can be individually addressed by the electric field generator (not shown).

In particular, each electrode in the electrode patch is electrically isolated from the others and is connected to the electric field generator by means of a single isolated electrical wire. The wires of all electrodes in an electrode patch are preferably grouped within a single cable.

However, it is not essentially that each (sub-)electrode is connected to the electric field generator by a respective wire. Rather, each (sub-)electrode may be connectable to the electric field generator using a same wire, e.g. which can be controllably disconnected from one sub-electrode and connected to another sub-electrode, e.g. under the control of a clinician or user. Thus, the sub-electrodes of the electrode patch are "individually addressable" in that it is possible to individually communicate with each sub-electrode.

The electrode patch 500 is formed of an array of N x M separate and electrically isolated (sub-)electrodes. In the illustrated example, the electrode patch is formed from an array of 4 x 4 (sub-)electrodes. In this example of Figure 5, a patch with 16 (sub-)electrodes is shown. However, a patch may have any number of (sub-)electrodes greater than or equal to 2 such as for example: 3, 4, 5, 6, 7, 8, 9, 10, 15, or 20. Although a squarely arranged (i.e. N x N) array of 4 by 4 electrodes is shown, other squarely arranged (such as 3 by 3 or 5 by 5) or non-squarely arranged (1 by 2, 2 by 4, 2 by 8 etc.) arrays or configurations may be used. A two-dimensional array of sub-electrodes on a patch is advantageous as it allows adjustment of field direction in multiple directions. For example, in Figure 5, a horizontal direction may be defined to run along sub-electrodes 512, 514, 515 and 516 and in Figure 7 (described in more detail below) from arm to arm. The direction of a field may be then be relayed "horizontally" by addressing different sub-electrodes of the patch in a row, and vertically by addressing different sub-electrodes in a column. Relay along angled directions such as diagonal is also possible and may include a change of electrode involving row and column number change such as with going from 512 to 510 or even 504 etc.

The real shape of an electrode (used in the electrode patch) may be different and may be different for all or some of the sub-electrodes. For example, one or more electrodes may be rectangular, square, triangular, circular or any other shape. Preferred shapes are those that are capable of close packing on substantially flat surfaces such as triangular shapes, tetrangular shapes, hexangular shapes or the like. Such embodiments may be advantageous for surface area use and to more/most efficiently and/or homogeneously generate the electric fields while having a good angle dependency of the field.

In use, at least one of the electrodes of the set of external electrodes comprises an electrode patch (such as the electrode patch 500 illustrated in Figure 5). The orientation/direction of an electric field (generated using the electrode patch) can be controlled by controlling which of the electrically responsive portions of the electrode patch are controlled by the electric field generator to define the electric field. This facilitates a degree of control over the direction of the electric field, and thereby facilitates adjustments to the crossing electric fields (e.g. the orthogonality of the crossing electric fields) without necessitating movement of the external electrodes.

Thus, an electric field generator may operate in a plurality of different control configurations. Each control configuration defines which of the electrically responsive portions of each electrode patch is active in the generation of an electric field.

The control configuration may be changed, for instance, automatically, e.g. if each electrically responsive portion is connected via a respective dedicated wire to the electric field generator so that each electrically responsive portion can be separately addressed at a same time.

In other embodiments, the control configuration is changed manually, e.g. by a clinician or other user mechanically adjusting which electrically responsive portions of the electrode patch are connected to the electric field generator.

It will be appreciated that more than one electrically responsive portion of any given electrode patch made be active at a same time. For instance, a set of two or more electrically responsive portions may be used to together act as a single electrode for generating an electric field (e.g. to act as a source and/or drain). This allows a greater level of control over the position of the effective electrical center of the electrode patch, thereby providing a greater number of possible orientations for the electric field generated using the electrode patch.

A control configuration which makes use of multiple electrically responsive portions of an electrode patch also provides a larger coupling area (compared to using only a single electrically responsive portion) and hence a lower coupling resistance into the body over using a single electrode only.

Each (sub-)electrode may be individually attachable to the skin of the subject by means of a conducting glue, such as those commonly used for other body surface electrodes such as ECG electrodes.

The (sub-)electrodes may be attached to a carrier substrate of an electrode patch. The carrier substrate and/or the patch itself may be rigid, but preferably is flexible to be able to conform to a part of the body of a subject. To this end, a carrier material may comprise plastic material or cloth, for example, either in sheet or woven form. Preferably the carrier material is a non-conducting material such that its interference with the electrodes is reduced.

Figure 6 illustrates a use case scenario for electrode patches, in which a set 600 of external electrodes (for an electric field generating arrangement) comprises six electrodes, each of which is an electrode patch, such the electrode patch 500 described with reference to Figure 5.

This proposed approach facilitates control over the orientation/direction of at least three electric fields (e.g. respectively between three electrode pairs formed by the six external electrodes).

However, the skilled person will appreciate that any suitable combination of electrode patches and non-patch based electrodes can be used. For instance, in some examples, only one of the electrodes of each electrode pair need comprise an electrode patch to facilitate control over the direction of each induced electric field. In other examples, one of the electric fields may be non-adjustable (e.g. be formed between an electrode pair comprising two electrodes), with the other electric fields being generated using a respective electrode pair that comprises at least one electrode patch.

Figures 7 and 8 illustrates non-exhaustive examples of alternative configurations for an electrode patch.

Figure 7 illustrates an electrode patch 700 formed from a plurality of individually addressable (and electrically isolated) sub-electrodes, arranged in the shape of a cross or plus-sign (i.e. "+"). Thus, the sub-electrodes are formed in a plurality of "arms" around a center.

Figure 8 illustrates an electrode patch 800 formed from a plurality of individually addressable (and electrically isolated) sub-electrodes, arranged around the border of an oblong/rectangular shape.

Such shapes might be advantageous to reduce the amount of wires/cabling towards an electrode patch while still maintain a high level of control over which area is used, or adapt an electrode patch to a particular anatomical shape of the body.

Other arrangement of sub-electrodes may be triangular or circular, i.e. sub-electrodes may be positioned to form a triangular or circular shape.

Although the above described examples of electrode patches are formed from an array of electrically isolated sub-electrodes, this is not an essential feature of the invention.

For instance, instead of using multiple discrete (i.e. electrically isolated) sub-electrodes, a single sheet of electrically responsive material could be used. In this scenario, the location of the (single) wire-connection(s) to the sheet determines the direction and position of the center of gravity of the electrical current lobe through the body, i.e. the position of the electrically responsive portion of the sheet. Mechanical connection of the wire(s) can be a needle/screw/push connection to the patch material.

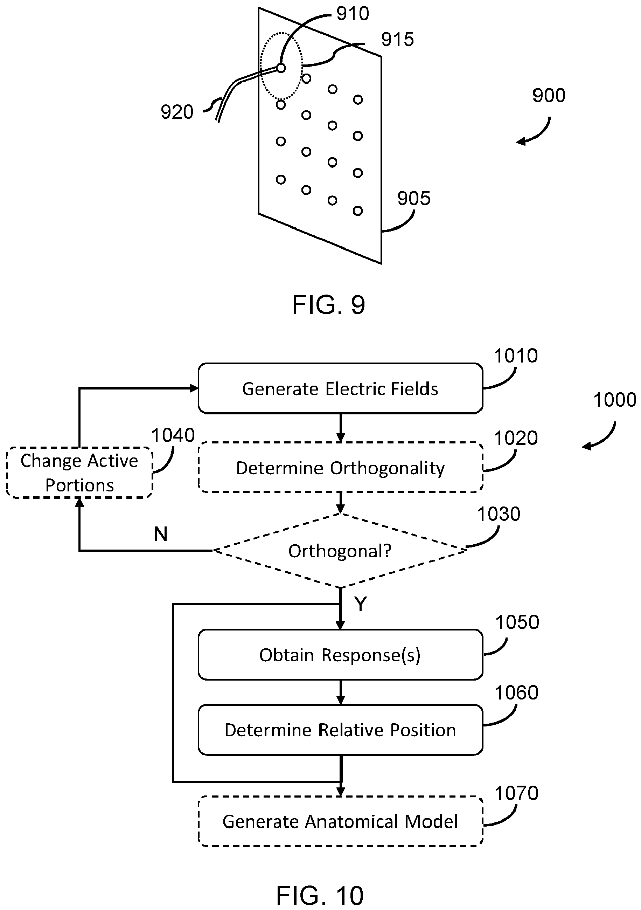

Figure 9 illustrates an example of an electrode patch 900 that is formed of a single sheet 905 of electrically responsive material (i.e. partly conductive material). A position 910 at which the (single) wire 920 is connected to the sheet 905 defines the position of the electrically responsive portion 915 of the sheet 905. The illustrated electrically responsive portion is schematically illustrated for improved understanding, but the skilled person will appreciate that the size and/or shape of the electrically responsive portion will depend upon the nature/material/structure of the sheet 905.

Figure 9 also illustrates a number of alternative connection positions for a wire 920, where the position of a connection defines the positions of the electrically responsive portions of the sheet 905. These are illustrated using circles depicting potential connection points for a wire.

Where the electrode patch is formed of a single sheet, different electrically responsive portions of the electrode patch are able to overlap another electrically responsive portion, and differ by differing positions of the mechanical connection of a wire with the sheet.

Preferably, the sheet has only a limited conductivity to be able to balance the current. For example, the sheet may be configured so that a resistance between the edge of the sheet and the center of the sheet is around the magnitude of the total patch-body-patch resistance, e.g. around 50Ω.

This approach adds some extra resistance, but has a limited (i.e. very small) effect on the accuracy of the device tracking system due to the use of electrical current excitation and high impedance (voltage) sensing using the internal electrodes.

The above description sets out how the proposed electric field generating arrangement can be used to control an orientation and/or direction of one or more crossing electric fields, e.g. to improve an orthogonality (being a measure of how close the electric fields are to being exactly orthogonal) of the crossing electrical fields.

In particular, different control configurations for the electric field generator can be used, each control configuration defining a different combination of which electrically responsive portions of the one or more electrode patches are used by the electric field generator to generate the crossing electric fields. Thus, different control configurations are associated with different "active" electrodes of the set of electrodes.

It would be advantageous if the most appropriate control configuration could be (automatically) selected, e.g. to provide crossing electric fields that have the best possible measure of orthogonality.

Figure 10 illustrates one example of determining in which control configuration to operate the electric field generator. This thereby demonstrates a (computer-implemented) method 1000 according to an embodiment, which may be carried out by the electric field generator and/or the device tracking arrangement.

The method 1000 comprises a step 1010 of generating crossing electric fields using a current control configuration of the electric field generator.

This step is performed, as previously described, by the electric field generator controlling the electric current provided to each of a plurality of electrodes of the set of electrodes positioned on the surface of the subject. The control configuration defines which of the electrically responsive portions of each electrode patch is addressed by the electric field generator (i.e. provided with a non-zero current and/or voltage). As previously noted, the direction of one or more of the at least two crossing electric fields differs in different control configurations

The method then performs a step 1020 of determining an orthogonality of the electric fields. In particular, step 1020 may comprise determining an orthogonality measure of the crossing electric fields.

Various mechanisms for determining an orthogonality (i.e. how close to orthogonal) of the electric fields are envisaged.

In one example, a measure of orthogonality between two electric fields can be performed as follows. Firstly, determine a response of the two electrically responsive portions of external electrodes (or set of electrically responsive portions) that contribute to a same electric field ("first electric field"), to another electric field ("second electric field"). Then, determine difference between these responses. This difference represents a measure of orthogonality between the first electric field and the second electric field. In particular, the smaller the difference, the closer the first electric field will be to being orthogonal to the second electric field. This approach does not necessitate the generation of the first electric field, and can therefore be used to predict an orthogonality between a potential electric field and an existing electric field.

Of course, a combination of differences (e.g. each representing an orthogonality between the first electric field and different other electric fields) could be used to determine an orthogonality of a plurality of electric fields. The combination may be an average and/or a sum.

In some examples, an orthogonality measure is obtained by obtaining, from one or more electrodes responsive to the at least two crossing electric fields, an electrical response of the one or more electrodes to the at least two crossing electric fields and determining, based on the electrical response of the one or more electrodes, a measure of orthogonality of the at least two crossing electric fields.

For the purposes of step 1020, these one or more electrodes may comprise any of the individually addressable internal and/or (electrically responsive portions of) external electrodes.

Determining a measure of orthogonality may be performed by generating a whitening or decorrelation transform. In particular, the weights of the whitening transform may be processed and/or used as the measure of orthogonality, as the value of the weights changes as the crossing electric fields converge to and diverge away from orthogonality.

The skilled person will appreciate that a whitening transform may be derived from a zero-phase component analysis, ZCA, of the responses of one or more electrodes. In particular, the whitening transform includes a multiplying step, performed on the data, with an appropriate matrix, the "whitening matrix". As a general rule, the closer the off-diagonal weights of the whitening matrix (which is related to the covariance matrix) are to zero, the closer the data can be considered to being orthogonal. As whitening might already be part of the processing chain for processing responses of internal electrodes (e.g. during generation of the mapping function), this approach can make use of already-available data to reduce processing time/complexity. These off-diagonal weights in the whitening matrix can reflect how much "effort" is put in to orthogonalization of the responses of the internal electrodes due to non-orthogonal electric fields. The less the (average or cumulative) size of these weights, the closer to orthogonal are the electric fields.

Another approach for determining a measure of orthogonality is to obtain a plurality of responses of one or more electrodes to each of at least two crossing electric fields and determining a correlation between the responses to each crossing electric field, e.g. using any known correlation technique (such as cross-correlation). The greater the determined correlation, the further the electric fields are from being orthogonal to one another.

To improve the accuracy of above-described approaches, it would be preferable if the plurality of responses are obtained at random or pseudorandom orientations and/or positions of the electrode(s) (and/or the interventional device on which they are mounted). This can be achieved, for instance, by providing (at a display) appropriate instructions to an operator of the electrodes and/or by only generating the measure of orthogonality if sufficient movement is detected (e.g. by an accelerometer or by tracking the predicted position of the electrodes).

Yet another approach could be to obtain a plurality of responses of two or more electrodes to each of at least two crossing electric fields and determining the difference between the electrical responses of said two or more electrodes for each of the crossing fields. The closer the difference between those responses is to zero in the subset of responses where the difference of the other field is largest, the closer the crossing fields are to orthogonality.

The method then performs a step 1030 of determining whether the orthogonality meets some predetermined criteria, e.g. if the measure of orthogonality has (or has not) reached/breached some predetermined threshold, in particular a threshold indicating an acceptable level of correlation (e.g. "minimal correlation").

In response to the orthogonality not meeting the predetermined criteria, then the method performs step 1040 of changing a control configuration of the electric field generator, and then reverting back to step 1010. The change to the control configuration may be to change the control configuration to a previously unused control configuration and/or according to some predetermined sequence of control configurations.

Step 1040 may be performed automatically (e.g. by the electric field generator) and/or manually.

Thus, a process of changing a control configuration of the electric field generator is iteratively repeated until the orthogonality of the electric fields meets some predetermined threshold.

Multiple instances of steps 1010-1040 may be performed at a same time, e.g. by multiplexing in frequency or time for a quick assessment of different control configurations.

In some examples, the method 1000 then performs a step 1050 of obtaining, at a first input and from an internal electrode positioned upon an interventional device within the subject, an electrical response of the internal electrode to the at least two crossing electric fields; and a step 1060 of determining, based on the electrical response of the internal electrode, a relative position of the interventional device and/or internal electrodes within the subj ect.

Thus, steps 1050 and 1060 of tracking (the position of) an interventional device based on the electrical responses of internal electrodes (of the interventional device) to the generated crossing electric fields may be performed.

The method 1000 may also comprise a step 1070 of generating an anatomical model of an anatomical cavity based on the tracked position of the interventional device, e.g. using any previously described imaging approach.

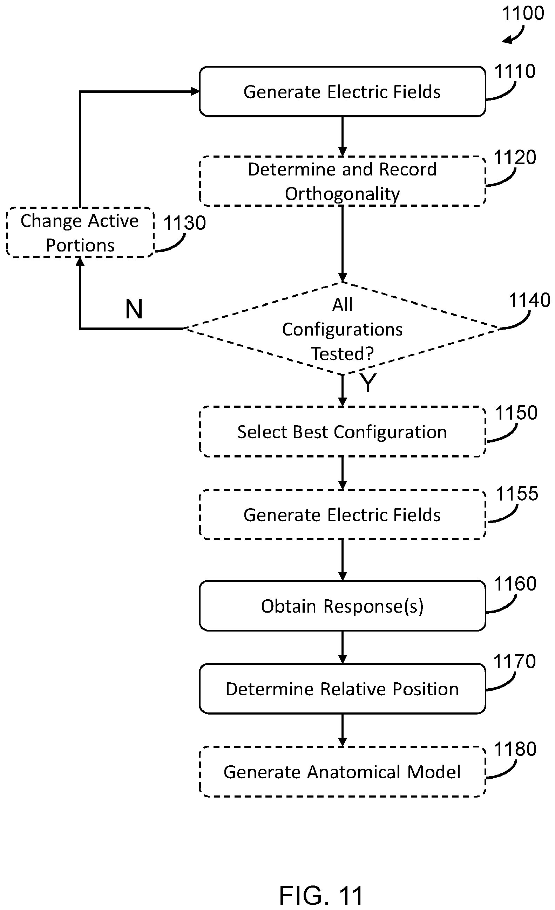

Figure 11 illustrates another example of determining in which control configuration to operate the electric field generator. This thereby demonstrates a (computer-implemented) method 1100 according to an embodiment, which may be carried out by the electric field generator and/or the device tracking arrangement.

The method 1100 comprises a step 1110 of generating crossing electric fields using a current control configuration of the electric field generator. This step may be effectively identical to the step 1010 previously described.

The method 1100 then performs a step 1120 of determining and recording an orthogonality of the crossing electric fields, e.g. using a previously described approach such as that disclosed in step 1020. This recorded orthogonality may be stored alongside an indicator of the control configuration (for the electric field generator) that resulted in the recorded orthogonality.

The method 1100 will then change the control configuration of the electric field generator, e.g. in a similar manner to previously described step 1040. The method then reverts back to step 1110.

The iterative sequence of steps 1110 to 1130 is repeated until all control configurations (e.g. in a predetermined sequence of control configurations and/or all possible control configurations) are tested. This is illustrated by decision step 1140, which takes place between steps 1120 and 1130.

Multiple instances of steps 1110 to 1140 may be performed at a same time, e.g. by multiplexing in frequency or time for a quick assessment of the different control configurations.

The method then performs a step 1150 of selecting the control configuration having the closest to orthogonal crossing electric fields, e.g. by identifying the recorded orthogonality that indicates the closest to orthogonal electric field arrangement, and controlling the electric field generator to operate in the selected control configuration.

The method may then perform a step 1155 of generating the electric fields using the selected control configuration for the electric field generator.

In some examples, the method 1100 then performs a step 1160 of obtaining, at a first input and from an internal electrode positioned upon an interventional device within the subject, an electrical response of the internal electrode to the at least two crossing electric fields; and a step 1170 of determining, based on the electrical response of the internal electrode, a relative position of the interventional device and/or internal electrodes within the subj ect.

Thus, steps 1160 and 1170 of tracking (the position of) an interventional device based on the electrical responses of internal electrodes (of the interventional device) to the generated crossing electric fields may be performed.

The method 1100 may also comprise a step 1180 of generating an anatomical model of an anatomical cavity based on the tracked position of the interventional device, e.g. using any previously described imaging approach.

The methods 1000 and 1100 can be carried out by the electric field generator and/or another processing system (e.g. a processor).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

The disclosed methods are preferably computer-implemented methods, and may be carried out by a suitable processing system. Any herein described processing system may be appropriately adapted to carry out any herein described method, just as any herein described method may be adapted to perform a process carried out by any herein described processing system.

There is also provided a computer program product comprising computer program code means which, when executed on a computing device having a processing system, cause the processing system to perform all of the steps of any herein described method.

A single processor or other unit may fulfill the functions of several items recited in the claims. A computer program may be stored/distributed on a suitable medium, such as an optical storage medium or a solid-state medium supplied together with or as part of other hardware, but may also be distributed in other forms, such as via the Internet or other wired or wireless telecommunication systems.

The mere fact that certain measures are recited in mutually different dependent claims does not indicate that a combination of these measures cannot be used to advantage. If the term "adapted to" is used in the claims or description, it is noted the term "adapted to" is intended to be equivalent to the term "configured to". Any reference signs in the claims should not be construed as limiting the scope.

## Claims

1. An electrode patch (500, 700, 800, 900) configured for use with an electric field generating arrangement (310) for inducing two or more crossing electric fields within a subject (190, 305) for use in tracking the position of an interventional device (335) within the subject, the electrode patch comprising:
two or more electrically responsive portions (501, 502, 503, 504, 505, 506, 507, 508, 509, 510, 511, 512, 513, 514, 515, 516, 915) that are individually addressable by an electric field generator (330) of the electric field generating arrangement, so that, when the electrode patch is positioned on the surface of the subject, a direction of an electric field between the electrode patch and one other electrode positioned on the surface of the subject is adjustable.

2. The electrode patch (500, 700, 800) of claim 1, wherein the electrode patch comprises an array of sub-electrodes electrically isolated from one another.

3. The electrode patch (500, 700, 800) of claim 2, wherein each electrically responsive portion comprises a different set of one or more of the sub-electrodes.

4. The electrode patch (900) of claim 1, wherein the electrode patch comprises a sheet of electrically responsive material, wherein each electrically responsive portions is able to overlap another electrically responsive portion.

5. A set (600) of external electrodes (321, 322, 323, 324, 325, 327) for positioning on a surface of the subject, the set of external electrodes comprising at least one electrode patch (500, 700, 800, 900) according to any of claims 1 to 4.

6. The set of external electrodes (600) of claim 5, wherein the at least one electrode patch comprises a plurality of electrode patches.

7. An electric field generator (330) for use with an electric field generating arrangement (310) for inducing two or more crossing electric fields within a subject (190, 305) for use in tracking the position of an interventional device (335) within the subject, the electric field generator being configured to control an electric signal provided to a set of external electrodes, as claimed in any of claims 5 or 6, to thereby, when the set of external electrodes is positioned on the surface of the subject, induce at least two crossing electric fields within the subject.

8. The electric field generator of claim 7 wherein the at least two crossing electric fields comprises three crossing electric fields.

9. The electric field generator (330) of any of claims 7 or 8, wherein the electric field generator (330) is further configured to determine a measure of orthogonality of the at least two electric fields for one or more different control configurations of the set of external electrodes.

10. The electric field generator (330) of claim 9, wherein the electric field generator is configured to determine an orthogonality of the at least two electric fields by:
obtaining, from one or more electrodes responsive to the at least two crossing electric fields, an electrical response of the one or more electrodes to the at least two crossing electric fields; and
determining, based on the electrical response of the one or more electrodes, a measure of orthogonality of the at least two crossing electric fields.

11. The electric field generator (330) of claim 9 or 10, wherein the electric field generator is configured to:
determine a measure of orthogonality of the at least two crossing electric fields for each of a plurality of different control configurations; and
identify the control configuration associated with the greatest orthogonality based on the determined measures of orthogonality.

12. An electric field generating arrangement (310), comprising:
the set of electrodes as claimed in any of claims 5 or 6; and
the electric field generator as claimed in any of claims 7 to 11.

13. A device tracking arrangement (300) comprising:
the electric field generating arrangement (310) of claim 12; and
a device tracking system (390) configured to:
obtain, at a first input and from an internal electrode (331, 332, 333) positioned upon an interventional device (325) within the subject (305), an electrical response of the internal electrode to the at least two crossing electric fields; and
determine, based on the electrical response of the internal electrode, a relative position of the interventional device within the subject.

14. A computer-implemented method (1000, 1100) for inducing two or more crossing electric fields within a subject (305) for use in tracking the position of an interventional device (335) within the subject, the computer-implemented method comprising:
controlling (1010, 1110, 1155) electric signals provided to each external electrode in a set (600) of external electrodes (321, 322, 323, 324, 325, 327) positioned on a surface of the subject to thereby induce at least two crossing electric fields within the subject,
wherein the set of external electrodes comprises at least one electrode patch (500, 700, 800, 900), each electrode patch comprising two or more electrically responsive portions (501, 502, 503, 504, 505, 506, 507, 508, 509, 510, 511, 512, 513, 514, 515, 516, 915) that are individually addressable so that, when the set of external electrodes is positioned on the surface of the subject, a direction of an electric field between the electrode patch and one other external electrode is adjustable.

15. A computer program product comprising computer program code means which, when executed on a computing device having a processing system, cause the processing system to perform all of the steps of the method (1000, 1100) according to claim 14.
